# EUROPEAN PATENT APPLICATION

(11) **EP 0 919 620 A2**
(43) Date of publication of application: **02.06.1999**
(21) Application number: 98309670.2
(22) Date of filing: 25.11.1998
(51) Int. Cl.: C12N 15/12, A61K 38/17, G01N 33/68, C12N 1/21, C07K 14/705, A61K 38/19, C12N 15/19, C07K 14/52

(54) **TRAILLK-3: a member of the TNF ligand family**

(30) Priority: 26.11.1997 US 66576 P; 11.08.1998 US 96171 P
(71) Applicant: ELI LILLY AND COMPANY, Indianapolis, Indiana 46285 (US)
(72) Inventor: Song, Ho Yeong, Carmel, Indiana 46032 (US)
(74) Representative: Denholm, Anna Marie

(57) **Abstract**

The invention provides isolated nucleic acid compounds, proteins and fragments thereof, said proteins being related to the family of TNF ligands. Also provided are vectors and transformed heterologous host cells for expressing the protein and a method for identifying compounds that bind and/or modulate the activity of said proteins, and a method for treating cancerous growths in a patient in need thereof.

SEQUENCE LISTING

<110> Song, Ho Yeong

<120> TNF Ligand Family Gene

<130> X11835

<140>
<141>

<160> 2

<170> PatentIn Ver. 2.0

<210> 1
<211> 1097
<212> DNA
<213> Homo sapiens

<220>
<221> CDS
<222> (359)..(1057)

<400> 1
gcctgttcct cctgggtgtc actggcagcc ctgtccttcc tagagggact ggaacctaat 60
tctcctgagg ctgagggagg gtggagggtc tcaaggcaac gctggcccca cgacggagtg 120
ccaggagcac taacagtacc cttagcttgc tttcctcctc cctccttttt attttcaagt 180
tcctttttat ttctccttgc gtaacaacct tcttcccttc tgcaccactg cccgtaccct 240
tacccgcccc gccacctcct tgctacccca ctcttgaaac cacagctgtt ggcagggtcc 300
ccagctcatg ccagcctcat ctcctttctt gctagccccc aaagggcctc caggcaac 358
atg ggg ggc cca gtc aga gag ccg gca ctc tca gtt gcc ctc tgg ttg 406
agt tgg ggg gca gct ctg ggg gcc gtg gct tgt gcc atg gct ctg ctg 454
acc caa caa aca gag ctg cag agc ctc agg aga gag gtg agc cgg ctg 502
cag ggg aca gga ggc ccc tcc cag aat ggg gaa ggg tat ccc tgg cag 550
agt ctc ccg gag cag agt tcc gat gcc ctg gaa gcc tgg gag agt ggg 598
gag aga tcc cgg aaa agg aga gca gtg ctc acc caa aaa cag aag aag 646
cag cac tct gtc ctg cac ctg gtt ccc att aac gcc acc tcc aag gat 694
gac tcc gat gtg aca gag gtg atg tgg caa cca gct ctt agg cgt ggg 742
aga ggc cta cag gcc caa gga tat ggt gtc cga atc cag gat gct gga 790
gtt tat ctg ctg tat agc cag gtc ctg ttt caa gac gtg act ttc acc 838
atg ggt cag gtg gtg tct cga gaa ggc caa gga agg cag gag act cta 886
ttc cga tgt ata aga agt atg ccc tcc cac ccg gac cgg gcc tac aac 934
agc tgc tat agc gca ggt gtc ttc cat tta cac caa ggg gat att ctg 982
agt gtc ata att ccc cgg gca agg gcg aaa ctt aac ctc tct cca cat 1030
gga acc ttc ctg ggg ttt gtg aaa ctg tgattgtgtt ataaaaagtg          1077
gctcccagct tggaagacca                                              1097
<210> 2
″
<211> 233
″
<212> PRT
″
<213> Homo sapiens
″

″
<400> 2

## Description

This application claims the benefit of U.S. Provisional Application No. 60/066,576, filed November 26, 1997, and U.S. Provisional Application No. 60/096,171, filed August 11, 1998.

This invention relates to recombinant DNA technology. In particular the invention pertains to a TNF ligand family gene, and its encoded protein. Also contemplated are methods for identifying compounds that bind said ligand, and methods for treating oncological disease in mammals.

Apoptosis (*i.e*. programmed cell death), is a process fundamental to the normal development and homeostasis of multicellular organisms. Deregulation of programmed cell death leads to a number of human diseases, including cancer, neurodegenerative disorders, and acquired immunodeficiency syndrome. The cell death machinery comprises effectors, activators, and negative regulators. Certain cytokines of the tumor necrosis factor (TNF) ligand family and their cognate receptors, including TNFR-1 and Fas (also known as Apo-1 or CD95), are classic triggers of the suicide response. TNF is the prototypic member of an emerging family of cytokines that function as prominent mediators of immune regulation and the inflammatory response. Eight other members of the TNF family are known, including lymphotoxin (Ltα, TNFβ), lymphotoxin β (LTβ), and ligands for CD40, CD30, CD27, OX40, 4-1BB, and Fas (APO-1). With one exception, all TNF ligands are type II membrane proteins, having homology at the C-terminal end. The exception, LTα, appears to be a secreted protein that also exists as a cell surface-associated member, termed LTβ. In addition, a proteolytically processed soluble form of TNF has been studied.

The TNF ligands interact with a parallel family of some twelve homologous receptors, characterized by cysteine-rich psuedorepeats in the extracellular region. As with the TNF ligands, the TNF receptors are variably expressed in a variety of cell types, including B cells, T cells, dendritic cells, and macrophages.

TNA and Fas ligand (also known as Apo-1L or CD95L) induce apoptosis by binding to receptors TNFR-1 and Fas. These receptors contain a domain (termed "death domain") that mediates the assembly of a signaling complex, leading to the recruitment of pro-apoptotic proteases.

Another member of the TNF ligand family, termed TRAIL (also known as Apo-2L), has been identified. Like the Fas ligand (FasL), TRAIL induces rapid apoptosis in transformed cell lines of diverse origin. Unlike FasL, whose transcripts are predominantly restricted to stimulated T cells, TRAIL expression is detected in many normal human tissues. This suggests that TRAIL is a member of the TNF ligand family that has marked pro-apoptotic potential for transformed cells. However, the inability of TRAIL to bind TNFR-1, Fas, or the recently identified death domain-containing receptor DR3 (also called Ws1-1, Apo-3, and TRAMP) suggests that TRAIL may interact with a yet unknown member of the TNF-receptor family.

The present invention provides isolated nucleic acid molecules and novel members of the TNF ligand family, termed herein "TRAILLK-3". Having the cloned TRAILLK-3 gene enables the production of recombinant protein, the isolation of orthologous genes from other organisms, and/or paralogous genes from the same organism, chromosome mapping studies, and the implementation of large scale screens to identify compounds that bind said receptor protein, as a means to identify potential pharmaceutical compounds for modulating the biological activity thereof. The proteins and peptides described herein are also useful therapeutic agents per se and for developing new compounds for treating cancer, and, among other things, as feed additives.

In one embodiment the present invention relates to an isolated nucleic acid molecule encoding TRAILLK-3 protein.

In another embodiment, the invention relates to a nucleic acid molecule comprising the nucleotide sequence identified as SEQ ID NO:1.

In another embodiment, the present invention relates to a nucleic acid that encodes SEQ ID NO:2, or fragment thereof.

In another embodiment, the present invention relates to a nucleic acid that is at least 75% identical to a nucleic acid that encodes SEQ ID NO:2, or fragment thereof.

In another embodiment, the present invention relates to a nucleic acid that hybridizes to SEQ ID NO:1 under high stringency conditions and encodes a protein that is capable of inducing apoptosis, and/or in treating cancer, and/or in preventing or inhibiting cancerous tumor growth, and/or in causing shrinkage of a cancerous tumor.

In another embodiment, the present invention relates to a nucleic acid that hybridizes to SEQ ID NO:1 under low stringency conditions and encodes a protein that is capable of inducing apoptosis and/or in treating cancer, and/or in preventing or inhibiting cancerous tumor growth, and/or in causing shrinkage of a cancerous tumor.

In another embodiment the present invention relates to an isolated protein molecule, or functional fragment thereof, wherein said protein molecule comprises the sequence identified as SEQ ID NO:2.

In yet another embodiment, the present invention relates to a recombinant DNA vector that incorporates the TRAILLK-3 gene (SEQ ID NO:1) in operable-linkage to gene expression sequences, enabling the gene to be transcribed and translated in a host cell.

In still another embodiment the present invention relates to host cells that have been transformed or transfected with the cloned TRAILLK-3 gene such that said gene is expressed in the host cell.

This invention also provides a method of determining whether a nucleic acid sequence of the present invention, or fragment thereof, is present within a nucleic acid-containing sample, comprising contacting the sample under suitable hybridization conditions with a nucleic acid probe of the present invention.

In a still further embodiment, the present invention relates to a method for treating cancer, and/or in preventing or inhibiting cancerous tumor growth, and/or in causing shrinkage of a cancerous tumor.

### Definitions

"Apoptosis" refers to the phenomenon of programmed cell death that is fundamental to the normal development and homeostasis of multicellular organisms. Deregulation of apoptosis leads to a number of human diseases, including cancer, neurodegenerative disorders, and AIDS. Certain cytokines of the TNF ligand family are triggers of apoptosis.

The terms "complementary" or "complementarity" as used herein refer to the capacity of purine and pyrimidine nucleotides to associate through hydrogen bonding to form double stranded nucleic acid molecules. The following base pairs are related by complementarity: guanine and cytosine; adenine and thymine; and adenine and uracil. As used herein, "complementary" means that the aforementioned relationship applies to substantially all base pairs comprising two single-stranded nucleic acid molecules over the entire length of said molecules. "Partially complementary" refers to the aforementioned relationship in which one of two single-stranded nucleic acid molecules is shorter in length than the other such that a portion of one of the molecules remains single-stranded.

"Conservative substitution" or "conservative amino acid substitution" refers to a replacement of one or more amino acid residue(s) in a protein or peptide as stipulated in Table 1.

"Fragment thereof" refers to a fragment, piece, or sub-region of a nucleic acid or protein molecule whose sequence is disclosed herein, such that the fragment comprises 5 or more amino acids, or 10 or more nucleotides that are contiguous in the parent protein or nucleic acid molecule. Fragment thereof may or may not retain biological activity. For example, a fragment of a protein disclosed herein could be used as an antigen to raise a specific antibody against the parent protein molecule. When referring to a nucleic acid molecule, "fragment thereof" refers to 10 or more contiguous nucleotides, derived from the parent nucleic acid, and also, owing to the genetic code, to the complementary sequence. For example if the fragment entails the sequence 5'-AGCTAG-3', then "fragment thereof" would also include the complementary sequence, 3'-TCGATC-5'.

The term "fusion protein" denotes a hybrid protein molecule not found in nature comprising a translational fusion or enzymatic fusion in which two or more different proteins or fragments thereof are covalently linked on a single polypeptide chain.

"Functional fragment" or "functionally equivalent fragment", as used herein, refers to a region, or fragment of a full length protein, or sequence of amino acids that, for example, comprises an active site, or any other conserved motif, relating to biological function. Functional fragments are capable of providing a substantially similar biological activity as a full length protein disclosed herein, *in vivo or in vitro, viz*. the capacity to promote apoptosis. Functional fragments may be produced by cloning technology, or as the natural products of alternative splicing mechanisms.

"Host cell" refers to any eucaryotic or procaryotic cell that is suitable for propagating and/or expressing a cloned gene contained on a vector that is introduced into said host cell by, for example, transformation or transfection, or the like.

TRAILLK-3 refers to a gene (SEQ ID NO:1) and a protein (SEQ ID NO:2) or peptide encoded thereby. TRAILLK-3 is a member of the family of TNF ligands. This family mediates a variety of biological effects including apoptosis, especially in tumor cells, and induction of the immune response.

The term "homolog" or "homologous" describes the relationship between different nucleic acid molecules or amino acid sequences in which said sequences or molecules are related by partial identity or similarity at one or more blocks or regions within said molecules or sequences.

The term "hybridization" as used herein refers to a process in which a single-stranded nucleic acid molecule joins with a complementary strand through nucleotide base pairing. "Selective hybridization" refers to hybridization under conditions of high stringency. The degree of hybridization depends upon, for example, the degree of homology, the stringency of hybridization, and the length of hybridizing strands.

"Isolated nucleic acid compound" refers to any RNA or DNA sequence, however constructed or synthesized, which is locationally distinct from its natural location.

A "nucleic acid probe" or "probe" as used herein is a labeled nucleic acid compound which hybridizes with another nucleic acid compound. "Nucleic acid probe" means a single stranded nucleic acid sequence that will combine with a complementary or partially complementary single stranded target nucleic acid sequence to form a double-stranded molecule. A nucleic acid probe may be an oligonucleotide or a nucleotide polymer. A probe will usually contain a detectable moiety which may be attached to the end(s) of the probe or be internal to the sequence of the probe.

The term "orthologue" or "orthologous" refers to two or more genes or proteins from different organisms that exhibit sequence homology.

The term "paralogue" or "paralogous" refers to two or more genes or proteins within a single organism that exhibit sequence homology.

The term "plasmid" refers to an extrachromosomal genetic element. The plasmids disclosed herein are commercially available, publicly available on an unrestricted basis, or can be constructed from readily available plasmids in accordance with published procedures.

A "primer" is a nucleic acid fragment which functions as an initiating substrate for enzymatic or synthetic elongation of, for example, a nucleic acid molecule.

The term "promoter" refers to a nucleic acid sequence that directs transcription, for example, of DNA to RNA. An inducible promoter is one that is regulatable by environmental signals, such as carbon source, heat, or metal ions, for example. A constitutive promoter generally operates at a constant level and is not regulatable.

"Recombinant DNA cloning vector" as used herein refers to any autonomously replicating agent, including, but not limited to, plasmids and phages, comprising a DNA molecule to which one or more additional DNA segments can or have been incorporated.

The term "recombinant DNA expression vector" or "expression vector" as used herein refers to any recombinant DNA cloning vector, for example a plasmid or phage, in which a promoter and other regulatory elements are present thereby enabling transcription of an inserted DNA, which may encode a protein.

The term "stringency" refers to hybridization conditions. High stringency conditions disfavor non-homologous base pairing. Low stringency conditions have the opposite effect. Stringency may be altered, for example, by temperature and salt concentration.

"Low stringency" conditions comprise, for example, a temperature of about 37° C or less, a formamide concentration of less than about 50%, and a moderate to low salt (SSC) concentration; or, alternatively, a temperature of about 50° C or less, and a moderate to high salt (SSPE) concentration, for example 1M NaCl.

"High stringency" conditions comprise, for example, a temperature of about 42° C or less, a formamide concentration of less than about 20%, and a low salt (SSC) concentration; or, alternatively, a temperature of about 65° C, or less, and a low salt (SSPE) concentration. For example, high stringency conditions comprise hybridization in 0.5 M NaHPO₄, 7% sodium dodecyl sulfate (SDS), 1 mM EDTA at 65°C (Ausubel, F.M. *et al.* Current Protocols in Molecular Biology, Vol. I, 1989; Green Inc. New York, at 2.10.3).

"SSC" comprises a hybridization and wash solution. A stock 20X SSC solution contains 3M sodium chloride, 0.3M sodium citrate, pH 7.0.

"SSPE" comprises a hybridization and wash solution. A 1X SSPE solution contains 180 mM NaCl, 9mM Na₂HPO₄, 0.9 mM NaH₂PO₄ and 1 mM EDTA, pH 7.4.

"Substantially pure," used in reference to a peptide or protein, means separation from other cellular and non-cellular molecules, including other protein molecules. A substantially pure preparation would be about at least 85% pure; preferably about at least 95% pure. A "substantially pure" protein can be prepared by a variety of techniques, well known to the skilled artisan, including, for example, the IMAC protein purification method.

"Treating" as used herein describes the management and care of a patient for the purpose of combating the disease, condition, or disorder and includes the administration of a protein of the present invention to prevent the onset of the symptoms or complications, alleviating the symptoms or complications, or eliminating the disease, condition, or disorder. Treating as used herein includes the administration of the protein for cosmetic purposes. A cosmetic purpose seeks to control, for example, the weight of a mammal to improve bodily appearance.

The term "vector" as used herein refers to a nucleic acid compound used for introducing exogenous or endogenous DNA into host cells. A vector comprises a nucleotide sequence which may encode one or more protein molecules. Plasmids, cosmids, viruses, and bacteriophages, in the natural state or which have undergone recombinant engineering, are examples of commonly used vectors.

The various restriction enzymes disclosed and described herein are commercially available and the manner of use of said enzymes including reaction conditions, cofactors, and other requirements for activity are well known to one of ordinary skill in the art. Reaction conditions for particular enzymes were carried out according to the manufacturer's recommendation.

The TRAILLK-3 gene encodes a novel, membrane-bound protein that is related to the TNF family. The TRAILLK-3 cDNA comprises a DNA sequence specified herein by SEQ ID NO:1. The coding region begins at base pair 359 and extends through base pair 1057 of SEQ ID NO:1. Those skilled in the art will recognize that owing to the degeneracy of the genetic code, numerous "silent" substitutions of nucleotide base pairs could be introduced into the sequence identified as SEQ ID NO:1 without altering the identity of the encoded amino acid(s) or protein product. All such substitutions are intended to be within the scope of the invention.

Also contemplated by the present invention are related proteins and related functional fragments such as, for example, smaller alternatively spliced forms. Related proteins comprise a genus in which amino acid substitutions of the primary sequence disclosed in SEQ ID NO:2 is altered by substitution, or replacement, or deletion, or insertion at one or more amino acid positions, such that TNF ligand function is maintained.

Functional fragments are conveniently identified as fragments of an intact TRAILLK-3 protein that retain the capacity to induce apoptosis.

Amino acid substitution modifications can be made in accordance with the following Table.

| ORIGINAL RESIDUE | EXEMPLARY SUBSTITUTIONS |
|---|---|
| ALA | SER |
| ARG | LYS |
| ASN | GLN; HIS |
| ASP | GLU |
| CYS | SER |
| GLN | ASN |
| GLU | ASP |
| GLY | PRO |
| HIS | ASN, GLN |
| ILE | LEU, VAL |
| LEU | ILE, VAL |
| LYS | ARG, GLN, GLU |
| MET | LEU, ILE |
| PHE | MET, LEU, TYR |
| SER | THR |
| THR | SER |
| TRP | TYR |
| TYR | TRP, PHE |
| VAL | ILE, LEU |

### Fragments of proteins

One embodiment of the instant invention provides fragments of the proteins disclosed that may or may not be biologically active. Such fragments are useful, for example, as an antigen for producing an antibody to said proteins.

Fragments of the proteins disclosed herein may be generated by any number of suitable techniques, including chemical synthesis of any portion of SEQ ID NO:2, proteolytic digestion of SEQ ID NO:2, or most preferably, by recombinant DNA mutagenesis techniques, well known to the skilled artisan. See. e.g. K. Struhl, "Reverse biochemistry: Methods and applications for synthesizing yeast proteins *in vitro," Meth. Enzymol.* 194, 520-535. For example, in a preferred method, a nested set of deletion mutations are introduced into the intact gene (SEQ ID NO:1) encoding TRAILLK-3 such that varying amounts of the protein coding region are deleted, either from the amino terminal end, or from the carboxyl end of the protein molecule. This method can also be used to create internal fragments of the intact protein in which both the carboxyl and amino terminal ends are removed. Several appropriate nucleases can be used to create such deletions, for example *Bal*31, or in the case of a single stranded nucleic acid molecule, mung bean nuclease. For simplicity, it is preferred that the TRAILLK-3 gene be cloned into a single-stranded cloning vector, such as bacteriophage M13, or equivalent. If desired, the resulting gene deletion fragments can be subcloned into any suitable vector for propagation and expression of said fragments in any suitable host cell.

The present invention also provides fragments of the proteins disclosed herein wherein said fragments retain receptor activity. As used herein, "functional fragments" refer to fragments of SEQ ID NO:2 that retain and exhibit, under appropriate conditions, measurable biological activity, for example, the capacity to induce apoptosis.

Functional fragments of the proteins disclosed herein may be produced as described above, preferably using cloning techniques to engineer smaller versions of the intact gene, lacking sequence from the 5' end, the 3' end, from both ends, or from an internal site. Fragments may be tested for biological activity using any suitable assay, for example, the ability of a protein fragment to induce apoptosis, *in vivo* or *in vitro.*

### Gene Isolation Procedures

Those skilled in the art will recognize that the TRAILLK-3 gene could be obtained by a plurality of recombinant DNA techniques including, for example, hybridization, polymerase chain reaction (PCR) amplification, or de novo DNA synthesis. (See *e.g*., T. Maniatis et *al.* Molecular Cloning: A Laboratory Manual, 2d Ed. Chap. 14 (1989)).

Methods for constructing cDNA libraries in a suitable vector such as a plasmid or phage for propagation in procaryotic or eucaryotic cells are well known to those skilled in the art. [See e.g. Maniatis et *al. Supra*]. Suitable cloning vectors are well known and are widely available.

The TRAILLK-3 gene or fragment thereof can be isolated a tissue in which said gene is expressed, for example, breast tissue. In one method, mRNA is isolated from a suitable tissue, and first strand cDNA synthesis is carried out. A second round of DNA synthesis can be carried out for the production of the second strand. If desired, the double-stranded cDNA can be cloned into any suitable vector, for example, a plasmid, thereby forming a cDNA library. Oligonucleotide primers targeted to any suitable region of SEQ ID NO:1 can be used for PCR amplification of TRAILLK-3. See e.g. PCR Protocols: A Guide to Method and Application, Ed. M. Innis et al., Academic Press (1990). The PCR amplification comprises template DNA, suitable enzymes, primers, and buffers, and is conveniently carried out in a DNA Thermal Cycler (Perkin Elmer Cetus, Norwalk, CT). A positive result is determined by detecting an appropriately-sized DNA fragment following agarose gel electrophoresis.

### Protein Production Methods

One embodiment of the present invention relates to the substantially purified protein encoded by the TRAILLK-3 gene.

Skilled artisans will recognize that the proteins of the present invention can be synthesized by a number of different methods, such as chemical methods well known in the art, including solid phase peptide synthesis or recombinant methods. Both methods are described in U.S. Patent 4,617,149, incorporated herein by reference.

The principles of solid phase chemical synthesis of polypeptides are well known in the art and may be found in general texts in the area. See, e.g., H. Dugas and C. Penney, Bioorganic Chemistry (1981) Springer-Verlag, New York, 54-92. For example, peptides may be synthesized by solid-phase methodology utilizing an Applied Biosystems 430A peptide synthesizer (Applied Biosystems, Foster City, CA) and synthesis cycles supplied by Applied Biosystems.

The proteins of the present invention can also be produced by recombinant DNA methods using the cloned TRAILLK-3 gene. Recombinant methods are preferred if a high yield is desired. Expression of the cloned gene can be carried out in a variety of suitable host cells, well known to those skilled in the art. For this purpose, the TRAILLK-3 gene is introduced into a host cell by any suitable means, well known to those skilled in the art. While chromosomal integration of the cloned gene is within the scope of the present invention, it is preferred that the gene be cloned into a suitable extra-chromosomally maintained expression vector so that the coding region of the TRAILLK-3 gene is operably-linked to a constitutive or inducible promoter.

The basic steps in the recombinant production of the TRAILLK-3 protein are:
a) constructing a natural, synthetic or semi-synthetic DNA encoding TRAILLK-3 protein;
b) integrating said DNA into an expression vector in a manner suitable for expressing the TRAILLK-3 protein, either alone or as a fusion protein;
c) transforming or otherwise introducing said vector into an appropriate eucaryotic or prokaryotic host cell forming a recombinant host cell,
d) culturing said recombinant host cell in a manner to express the TRAILLK-3 protein; and
e) recovering and substantially purifying the TRAILLK-3 protein by any suitable means, well known to those skilled in the art.

### Expressing Recombinant TRAILLK-3 Protein in Procaryotic and Eucaryotic Host Cells

Procaryotes may be employed in the production of recombinant TRAILLK-3 protein. For example, the *Escherichia coli* K12 strain 294 (ATCC No. 31446) is particularly useful for the prokaryotic expression of foreign proteins. Other strains of *E. coli,* bacilli such as *Bacillus subtilis,* enterobacteriaceae such as *Salmonella typhimurium* or *Serratia marcescans,* various Pseudomonas species and other bacteria, such as *Streptomyces,* may also be employed as host cells in the cloning and expression of the recombinant proteins of this invention.

Promoter sequences suitable for driving the expression of genes in procaryotes include b -lactamase [*e.g.* vector pGX2907, ATCC 39344, contains a replicon and b -lactamase gene], lactose systems [Chang et al., *Nature* (London), 275:615 (1978); Goeddel et al., *Nature* (London), 281:544 (1979)], alkaline phosphatase, and the tryptophan (trp) promoter system [vector pATH1 (ATCC 37695)], which is designed to facilitate expression of an open reading frame as a trpE fusion protein under the control of the trp promoter. Hybrid promoters such as the tac promoter (isolatable from plasmid pDR540, ATCC-37282) are also suitable. Still other bacterial promoters, whose nucleotide sequences are generally known, may be ligated to DNA encoding the protein of the instant invention, using linkers or adapters to supply any required restriction sites. Promoters for use in bacterial systems also will contain a Shine-Dalgarno sequence operably-linked to the DNA encoding the desired polypeptides. These examples are illustrative rather than limiting.

The proteins of this invention may be synthesized either by direct expression or as a fusion protein comprising the protein of interest as a translational fusion with another protein or peptide which may be removable by enzymatic or chemical cleavage. It is often observed in the production of certain peptides in recombinant systems that expression as a fusion protein prolongs the life span, increases the yield of the desired peptide, or provides a convenient means of purifying the protein. This is particularly relevant when expressing mammalian proteins in procaryotic hosts. A variety of peptidases (e.g. enterokinase and thrombin) which cleave a polypeptide at specific sites or digest the peptides from the amino or carboxy termini (*e.g*. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. *See e.g.,* P. Carter, "Site Specific Proteolysis of Fusion Proteins", Chapter 13, in Protein Purification: From Molecular Mechanisms to Large Scale Processes, American Chemical Society, Washington, D.C. (1990).

In addition to procaryotes, a variety of amphibian expression systems such as frog oocytes, and mammalian cell systems can be used. The choice of a particular host cell depends to some extent on the particular expression vector used. Exemplary mammalian host cells suitable for use in the present invention include HepG-2 (ATCC HB 8065), CV-1 (ATCC CCL 70), LC-MK₂ (ATCC CCL 7.1), 3T3 (ATCC CCL 92), CHO-K1 (ATCC CCL 61), HeLa (ATCC CCL 2), RPMI8226 (ATCC CCL 155), H4IIEC3 (ATCC CCL 1600), C127I (ATCC CCL 1616), HS-Sultan (ATCC CCL 1484), and BHK-21 (ATCC CCL 10), for example.

A wide variety of vectors are suitable for transforming mammalian host cells. For example, the pSV2-type vectors comprise segments of the simian virus 40 (SV40) genome required for transcription and polyadenylation. A large number of plasmid pSV2-type vectors have been constructed, such as pSV2-gpt, pSV2-neo, pSV2-dhfr, pSV2-hyg, and pSV2-b-globin, in which the SV40 promoter drives transcription of an inserted gene. These vectors are widely available from sources such as the American Type Culture Collection (ATCC), 12301 Parklawn Drive, Rockville, Maryland, 20852, or the Northern Regional Research Laboratory (NRRL), 1815 N. University Street, Peoria, Illinois, 61604.

Promoters suitable for expression in mammalian cells include the SV40 late promoter, promoters from eukaryotic genes, such as, for example, the estrogen-inducible chicken ovalbumin gene, the interferon genes, the glucocorticoid-inducible tyrosine aminotransferase gene, the thymidine kinase gene promoter, and the promoters of the major early and late adenovirus genes.

Plasmid pRSVcat (ATCC 37152) comprises portions of a long terminal repeat of the Rous Sarcoma virus, a virus known to infect chickens and other host cells. This long terminal repeat contains a promoter which is suitable for use in the vectors of this invention. H. Gorman *et al., Proc. Nat. Acad. Sci. (USA),* 79, 6777 (1982). The plasmid pMSVi (NRRL B-15929) comprises the long terminal repeats of the Murine Sarcoma virus, a virus known to infect mouse and other host cells. The mouse metallothionein promoter has also been well characterized for use in eukaryotic host cells and is suitable for use in the present invention. This promoter is present in the plasmid pdBPV-MMTneo (ATCC 37224) which can serve as the starting material for the construction of other plasmids of the present invention.

Transfection of mammalian cells with vectors can be performed by a plurality of well known processes including, but not limited to, protoplast fusion, calcium phosphate co-precipitation, electroporation and the like. *See*, *e.g.*, Maniatis *et al., supra.*

Some viruses also make appropriate vectors. Examples include the adenoviruses, the adeno-associated viruses, the vaccinia virus, the herpes viruses, the baculoviruses, and the rous sarcoma virus, as described in U.S. Patent 4,775,624, incorporated herein by reference.

Eucaryotic microorganisms such as yeast and other fungi are also suitable host cells. The yeast *Saccharomyces cerevisiae* is the preferred eucaryotic microorganism. Other yeasts such as *Kluyveromyces lactis* and *Pichia pastoris* are also suitable. For expression in *Saccharomyces,* the plasmid YRp7 (ATCC-40053), for example, may be used. *See, e.g., L.* Stinchcomb *et al., Nature,* 282, 39 (1979); J. Kingsman *et al., Gene,* 7, 141 (1979); S. Tschemper *et al., Gene,* 10, 157 (1980). Plasmid YRp7 contains the TRP1 gene which provides a selectable marker for use in a trpl auxotrophic mutant.

### Purification of Recombinantly-Produced TRAILLK-3 Protein

An expression vector carrying the cloned TRAILLK-3 gene is transformed or transfected into a suitable host cell using standard methods. Cells that contain the vector are propagated under conditions suitable for expression of the recombinant TRAILLK-3 protein. For Example, if the recombinant gene has been placed under the control of an inducible promoter, suitable growth conditions would incorporate the appropriate inducer. The recombinantly-produced protein may be purified from cellular extracts of transformed cells by any suitable means.

In a preferred process for protein purification, the TRAILLK-3 gene is modified at the 5' end to incorporate several histidine residues at the amino terminus of the TRAILLK-3 protein. This "histidine tag" enables a single-step protein purification method referred to as "immobilized metal ion affinity chromatography" (IMAC), essentially as described in U.S. Patent 4,569,794, which hereby is incorporated by reference. The IMAC method enables rapid isolation of substantially pure recombinant TRAILLK-3 protein starting from a crude extract of cells that express a modified recombinant protein, as described above.

### Production of Antibodies

The proteins of this invention and fragments thereof may be used in the production of antibodies. The term "antibody" as used herein describes antibodies, fragments of antibodies (such as, but not limited, to Fab, Fab', Fab₂', and Fv fragments), and chimeric, humanized, veneered, resurfaced, or CDR-grafted antibodies capable of binding antigens of a similar nature as the parent antibody molecule from which they are derived. The instant invention also encompasses single chain polypeptide binding molecules.

The production of antibodies, both monoclonal and polyclonal, in animals, especially mice, is well known in the art. *See,* e.g., C. Milstein, Handbook of Experimental Immunology, (Blackwell Scientific Pub., 1986); J. Goding, Monoclonal Antibodies: Principles and Practice, (Academic Press, 1983). For the production of monoclonal antibodies the basic process begins with injecting a mouse, or other suitable animal, with an immunogen. The mouse is subsequently sacrificed and cells taken from its spleen are fused with myeloma cells, resulting in a hybridoma that reproduces *in vitro.* The population of hybridomas is screened to isolate individual clones, each of which secretes a single antibody species, specific for the immunogen. Each antibody obtained in this way is the clonal product of a single B cell.

Chimeric antibodies are described in U.S. Patent No. 4,816,567, the entire contents of which is herein incorporated by reference. This reference discloses methods and vectors for the preparation of chimeric antibodies. An alternative approach is provided in U.S. Patent No. 4,816,397, the entire contents of which is herein incorporated by reference. This patent teaches co-expression of the heavy and light chains of an antibody in the same host cell.

The approach of U.S. Patent 4,816,397 has been further refined in European Patent Publication No. 0 239 400. The teachings of this European patent publication are a preferred format for genetic engineering of monoclonal antibodies. In this technology the complementarity determining regions (CDRs) of a human antibody are replaced with the CDRs of a murine monoclonal antibody, thereby converting the specificity of the human antibody to the specificity of a murine antibody.

Single chain antibodies and libraries thereof are yet another variety of genetically engineered antibody technology that is well known in the art. (See, e.g. R.E. Bird, et *al.,* Science 242:423-426 (1988); PCT Publication Nos. WO 88/01649, WO 90/14430, and WO 91/10737. Single chain antibody technology involves covalently joining the binding regions of heavy and light chains to generate a single polypeptide chain. The binding specificity of the intact antibody molecule is thereby reproduced on a single polypeptide chain.

The antibodies contemplated by this invention are useful in diagnostics, therapeutics or in diagnostic/therapeutic combinations.

The proteins of this invention or suitable fragments thereof can be used to generate polyclonal or monoclonal antibodies, and various inter-species hybrids, or humanized antibodies, or antibody fragments, or single-chain antibodies. The techniques for producing antibodies are well known to skilled artisans. (*See e.g.* A.M. Campbell, Monoclonal Antibody Technology: Laboratory Techniques in Biochemsitry and Molecular Biology, Elsevier Science Publishers, Amsterdam (1984); Kohler and Milstein, *Nature* 256, 495-497 (1975); Monoclonal Antibodies: Principles & Applications Ed. J.R.Birch & E.S. Lennox, Wiley-Liss, 1995.

A protein used as an immunogen may be modified or administered in an adjuvant, by subcutaneous or intraperitoneal injection into, for example, a mouse or a rabbit. For the production of monoclonal antibodies, spleen cells from immunized animals are removed, fused with myeloma cells, such as SP2/0-Ag14 cells, and allowed to become monoclonal antibody producing hybridoma cells in the manner known to the skilled artisan. Hybridomas that secrete a desired antibody molecule can be screened by a variety of well known methods, for example ELISA assay, western blot analysis, or radioimmunoassay (Lutz *et al. Exp. Cell Res.* 175, 109-124 (1988); Monoclonal Antibodies: Principles & Applications Ed. J.R.Birch & E.S. Lennox, Wiley-Liss, 1995).

For some applications labeled antibodies are desirable. Procedures for labeling antibody molecules are widely known, including for example, the use of radioisotopes, affinity labels, such as biotin or avidin, enzymatic labels, for example horseradish peroxidase, and fluorescent labels, such as FITC or rhodamine (See e.g. Enzyme-Mediated Immunoassay, Ed. T. Ngo, H. Lenhoff, Plenum Press 1985; Principles of Immunology and Immunodiagnostics, R.M. Aloisi, Lea & Febiger, 1988).

Labeled antibodies are useful for a variety of diagnostic applications. In one embodiment the present invention relates to the use of labeled antibodies to detect the presence of TRAILLK-3. Alternatively, the antibodies could be used in a screen to identify potential modulators of TRAILLK-3. For example, in a competitive displacement assay, the antibody or compound to be tested is labeled by any suitable method. Competitive displacement of an antibody from an antibody-antigen complex by a test compound such that a test compound-antigen complex is formed provides a method for identifying compounds that bind TRAILLK-3.

Other embodiments of the present invention comprise isolated nucleic acid sequences that encode SEQ ID NO:2, or related nucleic acids that are at least about 75% identical to SEQ ID NO:1, or to their complementary sequence, or nucleic acids that hybridize to SEQ ID NO:1 under low or high stringency conditions. Such sequences may come, for example, from paralogous or orthologous genes.

The TRAILLK-3 gene (viz. SEQ ID NO:1) and related nucleic acid molecules that encode SEQ ID NO:2, or functional fragments thereof, may be produced by chemical synthetic methods. The synthesis of nucleic acids is well known in the art. *See*, *e.g.,* E.L. Brown, R. Belagaje, M.J. Ryan, and H.G. Khorana, *Methods in Enzymology,* 68:109-151 (1979). Fragments of the DNA sequence corresponding to the TRAILLK-3 gene could be generated using a conventional DNA synthesizing apparatus, such as the Applied Biosystems Model 380A or 380B DNA synthesizers (Applied Biosystems, Inc., 850 Lincoln Center Drive, Foster City, CA 94404) using phosphoramidite chemistry, thereafter ligating the fragments so as to reconstitute the entire gene. Alternatively, phosphotriester chemistry may be employed to synthesize the nucleic acids of this invention. (*See, e.g.,* M.J. Gait, ed., Oligonucleotide Synthesis, A Practical Approach, (1984)).

In an alternative methodology, namely PCR, the DNA sequences disclosed and described herein, comprising, for example, a portion or all of SEQ ID NO:1 can be produced from a plurality of starting materials. For example, starting with a cDNA preparation (e.g. cDNA library) derived from a tissue that expresses the TRAILLK-3 gene, suitable oligonucleotide primers complementary to SEQ ID NO:1 or to any sub-region therein, are prepared as described in U.S. Patent No. 4,889,818, hereby incorporated by reference. Other suitable protocols for the PCR are disclosed in PCR Protocols: A Guide to Method and Applications, Ed. Michael A. Innis et al., Academic Press, Inc. (1990). Using PCR, any region of the TRAILLK-3 gene can be targeted for amplification such that full or partial length gene sequences may be produced.

The ribonucleic acids of the present invention may be prepared using polynucleotide synthetic methods discussed *supra,* or they may be prepared enzymatically, for example, using RNA polymerase to transcribe a TRAILLK-3 DNA template.

The most preferred systems for preparing the ribonucleic acids of the present invention employ the RNA polymerase from the bacteriophage T7 or the bacteriophage SP6. These RNA polymerases are highly specific, requiring the insertion of bacteriophage-specific sequences at the 5' end of the template to be transcribed. See, Maniatis *et al., supra.*

This invention also provides nucleic acids, RNA or DNA, that are complementary to SEQ ID NO:1 or SEQ ID NO:4, or fragment thereof.

### Nucleic Acid Probes

The present invention also provides probes and primers useful for a variety of molecular biology techniques including, for example, hybridization screens of genomic, subgenomic, or cDNA libraries, as well as hybridization against nucleic acids derived from cell lines or tissues that originate from drug-resistant tumors. Such hybridization screens are useful as methods to identify homologous and/or functionally related sequences from the same or other organisms. A nucleic acid compound comprising SEQ ID NO:1, or a complementary sequence thereof, or a fragment thereof, which is at least 14 base pairs in length, and which will selectively hybridize to human DNA or mRNA encoding TRAILLK-3 protein or fragment thereof, or a functionally related protein, is provided. Preferably, the 14 or more base pair compound is DNA. *See e.g.* B. Wallace and G. Miyada, "Oligonucleotide Probes for the Screening of Recombinant DNA Libraries," In *Meth. Enzym.,* 152, 432-442, Academic Press (1987).

Probes and primers can be prepared by enzymatic or recombinant methods, well known to those skilled in the art (*See e.g.* Sambrook et al. *supra*). A probe may be a single stranded nucleic acid sequence which is complementary in some particular degree to a nucleic acid sequence sought to be detected ("target sequence"). A probe may be labeled with a detectable moiety such as a radio-isotope, antigen, or chemiluminescent moiety. A description of the use of nucleic acid hybridization as a procedure for the detection of particular nucleic acid sequences is described in U.S. Patent No. 4,851,330 to Kohne, entitled "Method for Detection, Identification and Quantitation of Non-Viral Organisms."

Having the DNA sequence of the present invention allows preparation of relatively short DNA (or RNA) sequences having the ability to specifically hybridize to gene sequences disclosed herein. In these aspects, nucleic acid probes of an appropriate length are prepared. The ability of such nucleic acid probes to specifically hybridize to a polynucleotide encoding a TRAILLK-3 gene or related sequence lends particular utility in a variety of embodiments. Most importantly, the probes may be used in a variety of assays for detecting the presence of complementary sequences in a given sample.

In certain embodiments, it is advantageous to use oligonucleotide primers. The sequence of such primers is designed using a polynucleotide of the present invention for use in detecting, amplifying or mutating a defined segment of a gene or polynucleotide that encodes a TRAILLK-3 polypeptide using PCR technology.

Preferred nucleic acid sequences employed for hybridization studies, or assays, include probe molecules that are complementary to at least an about 14 to an about 70-nucleotide long stretch of a polynucleotide that encodes a TRAILLK-3 polypeptide, such as the nucleotide base sequences designated as SEQ ID NO:1. A length of at least 14 nucleotides helps to ensure that the fragment is of sufficient length to form a duplex molecule that is both stable and selective. Molecules having complementary sequences over stretches greater than 14 bases in length are generally preferred, though in order to increase stability and selectivity of the hybrid. One will generally prefer to design nucleic acid molecules having gene-complementary stretches of 25 to 40 nucleotides, 55 to 70 nucleotides, or even longer where desired. Such fragments may be readily prepared by, for example, directly synthesizing the fragment by chemical means, by application of nucleic acid reproduction technology, such as the PCR TM technology of U.S. Pat. No. 4,603,102, herein incorporated by reference, or by excising selected DNA fragments from recombinant plasmids containing appropriate inserts and suitable restriction enzyme sites.

The following guidelines are useful for designing probes with desirable characteristics. The extent and specificity of hybridization reactions are affected by a number of factors that determine the sensitivity and specificity of a particular probe, whether perfectly complementary to its target or not. The affect of various experimental parameters and conditions are well known to those skilled in the art.

First, the stability of the probe:target nucleic acid hybrid should be chosen to be compatible with the assay conditions. This may be accomplished by avoiding long A and T rich sequences, by terminating the hybrids with G:C base pairs and by designing a probe with an appropriate Tm (i.e. melting temperature). The melting profile, including the Tm of a hybrid comprising an oligonucleotide and target sequence, may be determined using a Hybridization Protection Assay. The probe should be chosen so that the length and % GC content result in a Tm about 2°-10° C higher than the temperature at which the final assay will be performed. The base composition of the probe is also a significant factor because G-C base pairs exhibit greater thermal stability as compared to A-T base pairs. Thus, hybridization involving complementary nucleic acids of higher G-C content will be more stable at higher temperatures.

The ionic strength and incubation temperature under which a probe will be used should also be taken into account. It is known that hybridization will increase as the ionic strength of the reaction mixture increases, and that the thermal stability of molecular hybrids will increase with increasing ionic strength. On the other hand, chemical reagents such as formamide, urea, DMSO and alcohols, which disrupt hydrogen bonds, increase the stringency of hybridization. Destabilization of hydrogen bonds by such reagents can greatly reduce the Tm. In general, optimal hybridization for synthetic oligonucleotide probes of about 10-50 bases in length occurs approximately 5° C below the melting temperature for a given duplex. Incubation at temperatures below the optimum may allow mismatched base sequences to hybridize and can therefore result in reduced specificity.

The length of the target nucleic acid sequence and, accordingly, the length of the probe sequence can also be important. In some cases, there may be several sequences from a particular region, varying in location and length, which will yield probes with the desired hybridization characteristics. In other cases, one sequence may be significantly better than another even though the one sequence differs merely by a single base. Finally, there can be intramolecular and intermolecular hybrids formed within a probe if there is sufficient self-complementarity. Such structures can be avoided through careful probe design. Computer programs are available to search for this type of interaction.

A probe molecule may be used for hybridizing to a sample suspected of possessing a TRAILLK-3 or TRAILLK-3-related nucleotide sequence. The hybridization reaction is carried out under suitable conditions of stringency.

Alternatively, such DNA molecules may be used in a number of techniques including their use as: (1) diagnostic tools to detect polymorphisms in DNA samples from a human or other mammal; (2) means for detecting and isolating homologs of TRAILLK-3 and related polypeptides from a DNA library potentially containing such sequences; (3) primers for hybridizing to related sequences for the purpose of amplifying those sequences; and (4) primers for altering the native TRAILLK-3 DNA sequences; as well as other techniques which rely on the similarity of the DNA sequences to those of the TRAILLK-3 DNA segments herein disclosed.

Once synthesized, oligonucleotide probes may be labeled by any of several well known methods. *See e.g.* Maniatis *et.al.,* Molecular Cloning (2d ed. 1989). Useful labels include radioisotopes, as well as non-radioactive reporting groups. Isotopic labels include H³, S³⁵, P³², I¹²⁵, Cobalt, and C¹⁴. Most methods of isotopic labeling involve the use of enzymes and include methods such as nick-translation, end-labeling, second strand synthesis, and reverse transcription. When using radio-labeled probes, hybridization can be detected by autoradiography, scintillation counting, or gamma counting. The detection method selected will depend upon the hybridization conditions and the particular radio isotope used for labeling.

Non-isotopic materials can also be used for labeling, and may be introduced internally into the sequence or at the end of the sequence. Modified nucleotides may be incorporated enzymatically or chemically, and chemical modifications of the probe may be performed during or after synthesis of the probe, for example, by the use of non-nucleotide linker groups. Non-isotopic labels include fluorescent molecules, chemiluminescent molecules, enzymes, cofactors, enzyme substrates, haptens or other ligands. In a preferred embodiment of the invention, the length of an oligonucleotide probe is greater than or equal to about 18 nucleotides and less than or equal to about 50 nucleotides. Labeling of an oligonucleotide of the present invention may be performed enzymatically using [³²P]-labeled ATP and the enzyme T4 polynucleotide kinase.

### Vectors

Another aspect of the present invention relates to recombinant DNA cloning vectors and expression vectors comprising the nucleic acids of the present invention. The preferred nucleic acid vectors are those which comprise DNA. The most preferred recombinant DNA vectors comprise the isolated DNA sequence, SEQ ID NO:1.

The skilled artisan understands that choosing the most appropriate cloning vector or expression vector depends upon a number of factors including the availability of restriction enzyme sites, the type of host cell into which the vector is to be transfected or transformed, the purpose of the transfection or transformation (e.g., stable transformation as an extrachromosomal element, or integration into the host chromosome), the presence or absence of readily assayable or selectable markers (e.g., antibiotic resistance and metabolic markers of one type and another), and the number of copies of the gene desired in the host cell.

Vectors suitable to carry the nucleic acids of the present invention comprise RNA viruses, DNA viruses, lytic bacteriophages, lysogenic bacteriophages, stable bacteriophages, plasmids, viroids, and the like. The most preferred vectors are plasmids.

When preparing an expression vector the skilled artisan understands that there are many variables to be considered, for example, whether to use a constitutive or inducible promoter. The practitioner also understands that the amount of nucleic acid or protein to be produced dictates, in part, the selection of the expression system. Regarding promoter sequences, inducible promoters are preferred because they enable high level, regulatable expression of an operably-linked gene. The skilled artisan will recognize a number of suitable promoters that respond to a variety of inducers, for example, carbon source, metal ions, and heat. Other relevant considerations regarding an expression vector include whether to include sequences for directing the localization of a recombinant protein. For example, a sequence encoding a signal peptide preceding the coding region of a gene is useful for directing the extracellular export of a resulting polypeptide.

The present invention also provides a method for constructing a recombinant host cell capable of expressing proteins comprising SEQ ID NO:2, said method comprising transforming or otherwise introducing into a host cell a recombinant DNA vector that comprises an isolated DNA sequence that encodes SEQ ID NO:2. A suitable host cell is any eucaryotic cell that can accomodate high level expression of an exogenously introduced gene or protein, and that will incorporate said protein into its membrane structure. Vectors for expression are those which comprise SEQ ID NO:1 or fragment thereof. Transformed host cells may be cultured under conditions well known to skilled artisans such that SEQ ID NO:2 is expressed, thereby producing a recombinant TRAILLK-3 protein in the recombinant host cell.

For the purpose of identifying compounds having utility as modifiers of apoptosis, it would be desirable to identify compounds that bind the TRAILLK-3 protein and/or modify its activity. For the purpose of identifying or developing inhibitors or other modifiers that, for example, activate or inhibit the proteins disclosed herein, it would be desirable to identify compounds that bind the TRAILLK-3 protein. A method for determining agents that bind the TRAILLK-3 protein comprises contacting the TRAILLK-3 protein with a test compound and monitoring binding by any suitable means.

The instant invention provides a screening system for discovering compounds that bind the TRAILLK-3 protein, said screening system comprising the steps of:
a) preparing TRAILLK-3 protein;
b) exposing said TRAILLK-3 protein to a test compound;
c) quantifying the binding of said compound to TRAILLK-3 protein by any suitable means.

Utilization of the screening system described above provides a means to determine compounds that may alter the biological function of TRAILLK-3. This screening method may be adapted to large-scale, automated procedures such as a PANDEX® (Baxter-Dade Diagnostics) system, allowing for efficient high-volume screening of potential therapeutic agents.

In such a screening protocol TRAILLK-3 is prepared as described herein, preferably using recombinant DNA technology. A test compound is introduced into a reaction vessel containing the TRAILLK-3 protein or fragment thereof. Binding of TRAILLK-3 by a test compound is determined by any suitable means. For example, in one method radioactively-labeled or chemically-labeled test compound may be used. Binding of the protein by the compound is assessed, for example, by quantifying bound label versus unbound label using any suitable method. Binding of a test compound may also be carried out by a method disclosed in U.S. Patent 5,585,277, which hereby is incorporated by reference. In this method, binding of a test compound to a protein is assessed by monitoring the ratio of folded protein to unfolded protein, for example by monitoring sensitivity of said protein to a protease, or amenability to binding of said protein by a specific antibody against the folded state of the protein.

The foregoing screening methods are useful for identifying a ligand of a TRAILLK-3 protein, as a lead to a pharmaceutical compound for modulating apoptosis. A ligand that binds TRAILLK-3, or related fragment thereof, is identified, for example, by combining a test ligand with TRAILLK-3 under conditions that cause the protein to exist in a ratio of folded to unfolded states. If the test ligand binds the folded state of the protein, the relative amount of folded protein will be higher than in the case of a test ligand that does not bind the protein. The ratio of protein in the folded versus unfolded state is easily determinable by, for example, susceptibility to digestion by a protease, or binding to a specific antibody, or binding to chaperonin protein, or binding to any suitable surface.

The present invention also provides a pharmaceutical composition comprising as the active agent a polypeptide compound represented by SEQ ID NO:2, or a pharmaceutically acceptable non-toxic salt thereof, and a pharmaceutically acceptable solid or liquid carrier. For example, compounds comprising TRAILLK-3 can be admixed with conventional pharmaceutical carriers and excipients, and used in the form of tablets, capsules, elixirs, suspensions, syrups, wafers, and the like. The compositions comprising TRAILLK-3 will contain from about 0.1% to 90% by weight of the active compound, and more generally from about 10% to 30%. The compositions may contain common carriers and excipients such as corn starch or gelatin, lactose, sucrose, microcrystalline cellulose, kaolin, mannitol, dicalcium phosphate, sodium chloride, and alginic acid. The compounds can be formulated for oral or parenteral administration.

Skilled artisans will recognize that IC₅₀ values are dependent on the selectivity of the compound tested. For example, a compound with an IC₅₀ which is less than 10 nM is generally considered an excellent candidate for drug therapy. However, a compound which has a lower affinity, but is selective for a particular target, may be an even better candidate. The skilled artisan will recognize that any information regarding the binding potential, inhibitory activity, or selectivity of a particular compound is useful toward the development of pharmaceutical products.

The following examples more fully describe the present invention. Those skilled in the art will recognize that the particular reagents, equipment, and procedures described are merely illustrative and are not intended to limit the present invention in any manner.

### EXAMPLE 1

### RT-PCR Amplification of TRAILLK-3 Gene from mRNA

A TRAILLK-3 gene is isolated by reverse transcriptase PCR (RT-PCR) using conventional methods. Total RNA from a tissue that expresses the TRAILLK-3 gene, is prepared using standard methods. First strand cDNA synthesis is achieved using a commercially available kit (SuperScript™ System; Life Technologies) by PCR in conjunction with specific primers directed at any suitable region of SEQ ID NO:1, for example, the ATG start codon at base pair 359 of SEQ ID NO:1 and the TGA stop codon at base pair position 1057 of SEQ ID NO:1.

Amplification is carried out by adding to the first strand cDNA (dried under vacuum) : 8 µl of 10X synthesis buffer (200 mM Tris-HCl, pH 8.4; 500 mM KCl, 25 mM MgCl₂, 1 ug/ul BSA) ; 68 µl distilled water; 1 µl each of a 10 uM solution of each primer; and 1 µl Taq DNA polymerase (2 to 5 U/µl). The reaction is heated at 94° C for 5 min. to denature the RNA/cDNA hybrid. Then, 15 to 30 cycles of PCR amplification are performed using any suitable thermal cycle apparatus. The amplified sample may be analyzed by agarose gel electrophoresis to check for an appropriately-sized fragment.

### EXAMPLE 2

### Production of a Vector for Expressing TRAILLK-3 in a Host Cell

An expression vector suitable for expressing TRAILLK-3 or fragment thereof in a variety of procaryotic host cells, such as *E. coli* is easily made. The vector contains an origin of replication (Ori), an ampicillin resistance gene (Amp) useful for selecting cells which have incorporated the vector following a tranformation procedure, and further comprises the T7 promoter and T7 terminator sequences in operable linkage to a TRAILLK-3 coding region. Plasmid pETllA (obtained from Novogen, Madison WI) is a suitable parent plasmid. pETllA is linearized by restriction with endonucleases NdeI and BamHI. Linearized pETllA is ligated to a DNA fragment bearing NdeI and BamHI sticky ends and comprising the coding region of the TRAILLK-3 gene as disclosed by SEQ ID NO:1 or fragment thereof.

The TRAILLK-3 gene used in this construction may be slightly modified at the 5' end (amino terminus of encoded protein) in order to simplify purification of the encoded protein product. For this purpose, an oligonucleotide encoding 8 histidine residues is inserted after the ATG start codon. Placement of the histidine residues at the amino terminus of the encoded protein serves to enable the IMAC one-step protein purification procedure.

### EXAMPLE 3

### Recombinant Expression and Purification of TRAILLK-3 Protein

An expression vector that carries an ORF encoding TRAILLK-3 or fragment thereof and which ORF is operably-linked to an expression promoter is transformed into *E. coli* BL21 (DE3) (*hsd*S *gal* lcIts857 *ind*1*Sam*7*nin*5*lac*UV5-T7gene 1) using standard methods. Transformants, selected for resistance to ampicillin, are chosen at random and tested for the presence of the vector by agarose gel electrophoresis using quick plasmid preparations. Colonies which contain the vector are grown in L broth and the protein product encoded by the vector-borne ORF is purified by immobilized metal ion affinity chromatography (IMAC), essentially as described in US Patent 4,569,794.

Briefly, the IMAC column is prepared as follows. A metal-free chelating resin (e.g. Sepharose 6B IDA, Pharmacia) is washed in distilled water to remove preservative substances and infused with a suitable metal ion [e.g. Ni(II), Co(II), or Cu(II)] by adding a 50mM metal chloride or metal sulfate aqueous solution until about 75% of the interstitial spaces of the resin are saturated with colored metal ion. The column is then ready to receive a crude cellular extract containing the recombinant protein product.

After removing unbound proteins and other materials by washing the column with any suitable buffer, pH 7.5, the bound protein is eluted in any suitable buffer at pH 4.3, or preferably with an imidizole-containing buffer at pH 7.5.

### EXAMPLE 4

### Tissue Distributuion of TRAILLK-3 mRNA

The presence of TRAILLK-3 mRNA in a variety of human tissues was analyzed by Northern analysis. Total RNA from different tissues or cultured cells was isolated by a standard guanidine chloride/phenol extraction method, and poly-A⁺ RNA was isolated using oligo(dT)-cellulose type 7 (Pharmacia). Electrophoresis of RNA samples was carried out in formaldehyde followed by capillary transfer to Zeta-Probe TM nylon membranes (Bio-Rad, Hercules, Calif.). SEQ ID NO:1 was the template for generating probes using a MultiPrime™ random priming kit (Amersham, Arlington Heights, Ill.). The efficiency of the labeling reaction was approximately 4 x 10¹⁰ cpm incorporated per µg of template. The hybridization buffer contained 0.5M sodium phosphate, 7% SDS (wt/vol), 1% BSA (wt/vol), and 1 mM EDTA. Prehybridization was carried out in hybridization buffer at 65° C for 2 h and ³²P-labeled probe was added and incubation continued overnight. The filters were washed in Buffer A (40 mM sodium phosphate pH 7.2, 5% SDS [wt/vol], 0.5% BSA [wt/vol], and 1 mM EDTA) at 65° C for 1 h, and then in Buffer B (40 mM sodium phosphate, pH 7.2, 1% SDS [wt/vol], and 1 mM EDTA) at 65° C for 20 min. The filters were air-dried and exposed to Kodak X-OMAT AR film at -80° C with an intensifying screen.

### EXAMPLE 5

### Detecting Ligands that Bind TRAILLK-3 Using a Chaperonin Protein Assay

The wells of an ELISA plate are coated with chaperonin by incubation for several hours with a 4 ug/ml solution of the protein in Tris-buffered Saline (TBS: 10 mM Tris-HCl, pH7.5, 0.2M NaCl). The plates are then washed 3 times with TBS containing 0.1% Tween-20 (TBST). Then, a mixture of TRAILLK-3 protein (sufficient amount to saturate about 50% of the binding sites on chaperonin) and test compound (10⁻⁹ to 10⁻⁵ M) in about 50 µl volume is added to each well of the plate for an incubation of about 60 minutes. Aliquots of the well solutions are then transferred to the wells of fresh plates and incubated for 60 minutes at room temperature, followed by 3 washes with TBST. Next, about 50 µl of an antibody specific for TRAILLK-3 plus 5% nonfat dry milk are added to each well for a 30 minute incubation at room temperature. After washing, about 50 µl of goat anti-rabbit IgG alkaline phosphatase conjugate at an appropriate dilution in TBST plus 5% nonfat dry milk are added to each will and incubated 30 minutes at room temperature. The plates are washed again with TBST and 0.1 ml of 1 mg/ml p-nitrophenylphosphate in 0.1% diethanolamine is added. Color development (proportional to bound alkaline phosphatase antibody conjugate) is monitored with an ELISA plate reader. When test ligand binding has occurred, ELISA analysis reveals TRAILLK-3 in solution at higher concentrations than in the absence of test ligand.

### EXAMPLE 6

### Production of an Antibody to TRAILLK-3 Protein

Substantially pure TRAILLK-3 protein or fragment thereof is isolated from transfected or transformed cells using any of the well known methods in the art, or by a method specifically disclosed herein. Concentration of protein in a final preparation is adjusted, for example, by filtration through an Amicon filter device such that the level is about 1 to 5 ug/ml. Monoclonal or polyclonal antibody can be prepared as follows.

Monoclonal antibody can be prepared from murine hybridomas according to the method of Kohler and Milstein (*Nature,* 256, 495, 1975), or a modified method thereof. Briefly, a mouse is repetitively inoculated with a few micrograms of the protein or fragment thereof, or fusion peptide thereof, over a period of a few weeks. The mouse is then sacrificed and the antibody producing cells of the spleen isolated. The spleen cells are fused by means of polyethylene glycol with mouse myeloma cells. Fused cells that produce antibody are identified by any suitable immunoassay, for example, ELISA, as described in E. Engvall, *Meth. Enzymol.,* 70, 419, 1980.

Polyclonal antiserum can be prepared by well known methods (See e.g. J. Vaitukaitis *et.al. Clin. Endocirnol. Metab.* 33, 988, 1971) that involve immunizing suitable animals with the proteins, fragments thereof, or fusion proteins thereof, disclosed herein. Small doses (e.g. nanogram amounts) of antigen administered at multiple intradermal sites appears to be the most reliable method.

### Annex to the description

SEQUENCE LISTING

SEQ ID NO:1

GCCTGTTCCTCCTGGGTGTCACTGGCAGCCCTGTCCTTCCTAGAGGGACTGGAACCTAA
TTCTCCTGAGGCTGAGGGAGGGTGGAGGGTCTCAAGGCAACGCTGGCCCCACGACGG
AGTGCCAGGAGCACTAACAGTACCCTTAGCTTGCTTTCCTCCTCCCTCCTTTTTATTTTCA
AGTTCCTTTTTATTTCTCCTTGCGTAACAACCTTCTTCCCTTCTGCACCACTGCCCGTACC
CTTACCCGCCCCGCCACCTCCTTGCTACCCCACTCTTGAAACCACAGCTGTTGGCAGGG
TCCCCAGCTCATGCCAGCCTCATCTCCTTTCTTGCTAGCCCCCAAAGGGCCTCCAGGCA
ACATGGGGGGCCCAGTCAGAGAGCCGGCACTCTCAGTTGCCCTCTGGTTGAGTTGGGG
GGCAGCTCTGGGGGCCGTGGCTTGTGCCATGGCTCTGCTGACCCAACAAACAGAGCTG
CAGAGCCTCAGGAGAGAGGTGAGCCGGCTGCAGGGGACAGGAGGCCCCTCCCAGAAT
GGGGAAGGGTATCCCTGGCAGAGTCTCCCGGAGCAGAGTTCCGATGCCCTGGAAGCCT
GGGAGAGTGGGGAGAGATCCCGGAAAAGGAGAGCAGTGCTCACCCAAAAACAGAAGAA
GCAGCACTCTGTCCTGCACCTGGTTCCCATTAACGCCACCTCCAAGGATGACTCCGATG
TGACAGAGGTGATGTGGCAACCAGCTCTTAGGCGTGGGAGAGGCCTACAGGCCCAAGG
ATATGGTGTCCGAATCCAGGATGCTGGAGTTTATCTGCTGTATAGCCAGGTCCTGTTTCA
AGACGTGACTTTCACCATGGGTCAGGTGGTGTCTCGAGAAGGCCAAGGAAGGCAGGAG
ACTCTATTCCGATGTATAAGAAGTATGCCCTCCCACCCGGACCGGGCCTACAACAGCTG
CTATAGCGCAGGTGTCTTCCATTTACACCAAGGGGATATTCTGAGTGTCATAATTCCCCG
GGCAAGGGCGAAACTTAACCTCTCTCCACATGGAACCTTCCTGGGGTTTGTGAAACTGT
GATTGTGTTATAAAAAGTGGCTCCCAGCTTGGAAGACCA

SEQ ID NO:2






## Claims

1. A substantially pure protein having the amino acid sequence which is SEQ ID NO: 2.

2. An isolated nucleic acid compound encoding the protein of Claim 1, said protein having the amino acid sequence that is SEQ ID NO: 2.

3. An isolated nucleic acid compound that is complementary to SEQ ID NO:1.

4. An isolated nucleic acid compound that hybridizes to SEQ ID NO:1 under low stringency conditions.

5. An isolated nucleic acid compound that encodes a protein having TNF family ligand activity wherein said nucleic acid hybridizes to SEQ ID NO:1 under high stringency conditions.

6. A vector comprising an isolated nucleic acid compound comprising SEQ ID NO:1.

7. A vector, as in Claim 6, wherein said isolated nucleic acid compound is operably-linked to a promoter sequence.

8. A host cell containing a vector of Claim 6.

9. A host cell containing a vector of Claim 7.

10. A method for constructing a recombinant host cell having the potential to express SEQ ID NO:2, said method comprising introducing into said host cell by any suitable means a vector of Claim 7.

11. A method for expressing SEQ ID NO:2 in a recombinant host cell of Claim 10, said method comprising culturing said recombinant host cell under conditions suitable for gene expression.

12. A method for identifying compounds that bind a protein identified herein as SEQ ID NO:2, comprising the steps of:
a) admixing a substantially purified preparation of a protein comprising SEQ ID NO:2 with a test compound; and
b) monitoring by any suitable means a binding interaction between said protein and said compound.

13. A nucleic acid molecule comprising a fragment of SEQ ID NO:1, wherein said fragment consists of at least 14 contiguous base pairs therefrom.

14. An antibody that selectively binds to a protein identified herein as SEQ ID NO:2.

15. A pharmaceutical formulation comprising as an active ingredient TRAILLK-3 protein, associated with one or more pharmaceutically acceptable carriers, excipients, or diluents thereof.

16. TRAILLK-3 or an analog thereof for use in treating cancer in a patient in need thereof.

17. A method of treating cancer in a patient in need thereof comprising administering an effective amount of a composition comprising as an active ingredient, TRAILLK-3.

18. A method, as in Claim 17, wherein said cancer is breast cancer.
